# EUROPEAN PATENT SPECIFICATION

(11) **EP 0 873 131 B1**
(45) Date of publication and mention of the grant of the patent: **08.09.2004**
(21) Application number: 96939357.8
(22) Date of filing: 20.11.1996
(51) Int. Cl.: A61K 35/78

(54) **PROCESSES FOR PREPARATION OF RG3 AND RG5 GINSENOSIDES**
VERFAHREN ZUR HERSTELLUNG VON RG3 UND RG5 GINSENOSIDEN
PROCEDES DE PREPARATION DES GINSENOSIDES RG3 ET RG5

(30) Priority: 22.11.1995 KR 9542997; 13.11.1996 KR 9653685
(43) Date of publication of application: 28.10.1998
(73) Proprietor: Cheil Je Dang Co., Seoul 100-095 (KR); Ginseng Science Inc., Seoul 151-742 (KR)
(72) Inventor: PARK, Man Ki, Shiwon Apartment 310-1502, Sungnam-shi, Kyungki-do 463-500 (KR); KIM, Nak Doo, Shindaebang 2nd Apartment 101-1101, Dongjak-ku, Seoul 156-010 (KR); PARK, Jeong Hill, Kangnam-ku, Seoul 135-239 (KR); KIM, Jong Moon, Songpa-ku, Seoul 138-222 (KR); KANG, Su, Yeon, Kwanak-ku, Seoul 151-012 (KR); KIM, Wang You, Songpa-ku, Seoul 138-160 (KR)
(74) Representative: Dost, Wolfgang, Dr.rer.nat., Dipl.-Chem.
(86) International application number: PCT/KR1996/000207
(87) International publication number: WO 1997/018824

(56) References cited:
- WO-A-96/40181
- FR-A- 2 671 488
- FR-A- 2 712 191
- PATENT ABSTRACTS OF JAPAN, Vol. 17, No. 270 (C-1063), 1993; & JP,A,05 009 123 (ISAO KITAGAWA).

## Description

### TECHNICAL FIELD

The present invention relates to a process for preparing ginsenoside Rg₃ and/or Rg₅, one of the saponin components of ginseng.

### BACKGROUND ART

Since ancient times, ginseng has been widely used as the most typical nutritive and tonics. Recently, many scientific results on the components and pharmacological effects of ginseng have been reported, thereby the mysterious pharmacological effects of ginseng have been come to light under modern science.

Ginseng has been generally used in the form of fresh ginseng as it is harvested after cultivation, white ginseng produced by drying the fresh ginseng at normal temperature, or red ginseng prepared by heating the fresh ginseng at 98 to 100°C. Among them, particularly, the red ginseng has been recognized as having much more potent pharmacological effect than white ginseng. Accordingly, recently many researches on the specific components in red ginseng have been actively conducted. It has been considered that the specific components are produced during the process for preparing red ginseng and can explain the excellent pharmacological effect of red ginseng.

Accordingly, a number of pharmacological effects attributed to ginseng extracts have previously been described.

The Patent Abstracts of Japan, Vol. 17, No. 270 (C-1063), 1993 and the Japanese Patent Application JP-A-05 009 123 (Isao Kitagawa) describe a carcinostatic agent containing ginsenoside Rg₃ obtained form Panax ginseng C.A. Meyer. Said agent is said to be capable of selectively suppressing the infiltration of various cancerous cells and of suppressing the metastatsis of cancerous cells.

The French Patent Application FR-A-2712191 refers to ginseng extracts prepared by alcoholic extraction having pharmacological effects on the circulatory system.

The French Patent Application FR-A-2671488 describes that ginseng extracts may be used on the cardio-vascular level for the treatment of pain, palpitations or troubles of the cardiac rhythm.

Recently, as described in the International Patent Application WO-A-9640181 we have found that processed ginseng has an enhanced pharmacological effect, in particular when produced by heating ginseng for 0.5 to 20 hours at 120 to 180°C.

### DISCLOSURE OF INVENTION

Thus, the present inventors have made a study of the means capable of enhancing the pharmacological effect of ginseng by increasing the specific components in red ginseng. As a result of such study, we have identified that a processed ginseng having a much more enhanced pharmacological effect than fresh ginseng, white ginseng or red ginseng can be prepared by heating ginseng for 0.5 to 20 hours at a high temperature of 110 to 180°C. In the course of separating the various components present in such a processed ginseng and determining the pharmacological effects thereof, the present inventors have also identified that ginsenoside Rg₃ and Rg₅ which are not contained or contained in a minor amount in white ginseng or red ginseng and of which the pharmacological effect has been substantially not known, exhibit an excellent vasodilation effect. Thus, we have completed the present invention.

Therefore, the present invention relates to a process preparing ginsenoside Rg₃ and/or Rg₅.

### BRIEF DESCRIPTION OF DRAWINGS

For a thorough understanding of the nature and objects of the invention, reference should be made to the following detailed description taken in connection with the accompanying drawings in which :
Figure 1 represents a vasodilation of ginsenoside Rg₃ which is administered alone in the presence of endothelium (- ○ -) in comparison with vasodilations when ginsenoside Rg₃ is administered after removing endothelium(-○-); and administered with 10⁻⁶M of methylene blue(MB) (-Δ-) and with 10⁻⁶M of nitro-L-arginine(NLA) (- ▲ -) in the presence of endothelium;
Figure 2 represents vasodilation effects when the processed panaxadiol-based saponin fraction prepared in Example 7 is administered in the presence of endothelium (- ● -) and after removing endothelium(- ○ -);
Figure 3 represents survival ratio of HUVEC cells when the cells are cultured with dimethylsulfoxide (DMSO) (- ○ -) and with ginsenoside Rg₃-DMSO solution(- ▼ -), as an index of the cytotoxicities to HUVEC cell of ginsenoside Rg₃ and DMSO used as a solvent;
Figure 4 represents a vasodilation of ginsenoside Rg₅ which is administered alone in the presence of endothelium (-○-) in comparison with vasodilations when ginsenoside Rg₅ is administered after removing endothelium(- **■** -); and administered with 10⁻⁶M of methylene blue (MB) (-**▼**-) and with 10⁻⁶M of nitro-L-arginine(NLA)(- Δ -) in the presence of endothelium; and
Figure 5 represents survival ratio of HUVEC cells when the cells are cultured with distilled water (- ● -) and with ginsenoside Rg₅-distilled water solution(-■-), as an index of the cytotoxicities to HUVEC cell of ginsenoside Rg₅ and distilled water used as a solvent;

### BEST MODE FOR CARRYING OUT THE INVENTION

The ginsenoside Rg₃ and Rg₅ prepared according to the present invention are saponin compounds having the following formulas. In the present invention, pure ginsenoside Rg₃ and/or Rg₅, processed ginseng rich in ginsenoside Rg₃ and/or Rg₅ components or an extract therefrom can be used as the source of ginsenoside Rg₃ and Rg₅.

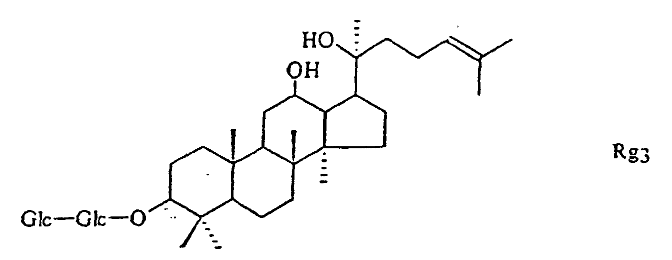

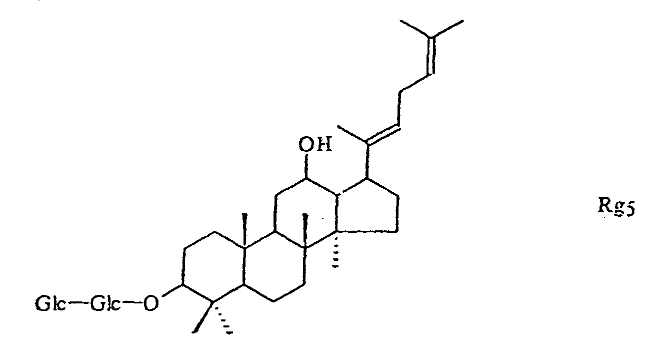

Ginseng extract rich in ginsenoside Rg₃ and/or Rg₅ can be obtained by the method wherein plant of *Panax* genus containing ginseng saponins, for example, Panax *ginseng, Panax notoginseng, Panax quinquefolium, Panax japonicus,* etc., leaves or tissue cultures thereof, or extract therefrom with water or lower alcohol are heated for 0.5 to 20 hours at a temperature of 110 to 180°C; the processed ginseng thus obtained is extracted with water or a suitable organic solvent such as methanol, ethanol, or a solvent mixture thereof, the extract is concentrated under reduced pressure, the residue is suspended in water, and then the suspension is extracted again with a nonpolar organic solvent such as hexane, ether, dichloromethane, chloroform, ethylacetate or a solvent mixture thereof; the remaining aqueous layer is extracted again with a polar organic solvent such as butanol and then the extract is subjected to chromatography to obtain a fraction containing Rg₃ or Rg₅, or a fraction containing Rg₃ and Rg₅ simultaneously. By repeatedly carrying out the chromatography procedure, the content of ginsenoside Rg₃ or Rg₅ can be more increased. Then, the fraction having a high content can be crystallized from a suitable solvent system such as water, lower alcohol, lower ketone, chloroform or a solvent mixture thereof to obtain pure ginsenoside Rg₃ or Rg₅. In this method, during the procedure of heat-treatment of ginseng, a sugar moiety attached to the 20th carbon of panaxadiol saponins present in ginseng such as ginsenosides Ra, Rb₁, Rb₂, Rc, Rd, etc. is removed to produce the desired ginsenoside Rg₃, and a double bond can be subsequently produced at the 20-position of Rg₃ by dehydration reaction to produce the desired ginsenoside Rg₅. Alternatively, the same results can be obtained in this process by inverting the order of the heat-treatment step and extraction step with organic solvent, or by heating ginseng under mild conditions, for example at 30 to 100°C, preferably at 70°C, simultaneously with carrying out acid- treatment with diluted inorganic acid such as hydrochloric acid, nitric acid, perchloric acid, etc., or lower organic acid such as acetic acid, tartaric acid, oxalic acid, etc., instead of heating at a high temperature, if necessary.

In addition, the same result can be obtained by directly heating or acid-hydrolyzing the known ginsenoside compounds Ra, Rb₁, Rb₂, Rc, Rd, etc., or a fraction thereof, instead of ginseng itself, according to the same manner as mentioned above.

In preparing ginseng extract containing Rg₃ and/or Rg₅, a fraction containing panaxadiol saponin component in a high concentration, which is prepared by extracting ginseng with an organic solvent, for example, alcohols such as methanol, ethanol, butanol, etc., ethers, or a solvent mixture thereof before heat-treatment to remove the undesired panaxatriol saponin components, can be subjected to heat-treatment according to the same manner as mentioned above to obtain a processed panaxadiol saponin fraction which contains Rg₃ and/or Rg₅ in large quantities and does not contain the undesired panaxatriol saponin component. The fraction thus obtained can be used as the processed ginseng extract containing Rg₃ and/or Rg₅ according to the present invention.

Since compositions containing ginsenoside Rg₃ and/or Rg₅ as prepared according to the present invention have potent vasodilation effects, they can be effectively used as agents for prevention or treatment of disorders caused by circulatory disturbances such as hypertension, arteriosclerosis, blood circulation disturbance, diabetes mellitus sexual disturbance, memory disturbance, etc. When a composition is used for such purposes in the clinical field, it can be combined with pharmaceutically acceptable carriers to prepare various formulations conventionally used in the pharmaceutical field, for example, oral preparations such as tablets, capsules, troches, solutions, suspensions, etc.; injectable preparations in the form of injectable solutions or suspensions or ready-to-use injectable dried powder which can be re-constituted with injectable distilled water just before injection, etc.; or locally applicable preparations such as ointments, creams, solutions, etc.

The carriers which can be used in such compositions are conventional ones in the pharmaceutical field, for example, binders, lubricants, disintegrating agents, excipients, solubilizers, dispersing agents, stabilizers, suspending agents, coloring agents, flavors and the like in the case of oral preparations; preservatives, agents for painlessness, solubilizers, stabilizers and the like in the case of injectable preparations; bases, excipients, lubricants, preservatives and the like in the case of locally applicable preparations. The pharmaceutical agents thus produced can be administered orally, or parenterally such as for example intravenously, peritoneally, subcutaneously, or can be topically applied. In addition, the oral preparations may be administered together with an antacid or in the form of an enteric-coated preparation which is formulated by covering the orally administrable solid preparation such as tablet with the enteric coatings, in order to prevent decomposition of the preparation by gastric acid when it is administered per orally.

Although the administration dosage to a human being of the ginsenoside Rg₃ and/or Rg₅ can be selected depending on the absorption, inactivating rate and excretion rate of the active component in the body, age, sex and condition of the subject patient, severity of the disorders to be treated and the like, it is generally administered to an adult in an amount of 5 to 500mg, preferably 10 to 200mg, daily. Therefore, when the composition of the present invention is formulated into the dosage unit form, each of the dosage unit form can contain ginsenoside Rg₃ and/or Rg₅ in an amount of 5 to 500mg, preferably 10 to 200mg considering the effective amount range as mentioned above. If necessary, the dosage unit form thus formulated can be administered using a specialized method according to the judgement of the specialist who arranges or observes the administration and the requirement of the individuals. The total daily dosage can also be divided into several portions and administered over several times, preferably 1 to 6 times.

As established by the test results as described below, since ginsenoside Rg₃ and Rg₅, as active ingredients of compositions, exhibit a vasodilation without any damage to vascular endothelium, they show no side effect such as hemolysis or toxicity against fishes and no acute toxicity against test animals, therefore they can be used safely.

The present invention is more specifically explained by the following examples, test examples and composition examples. However, it should be understood that the present invention is not limited to those examples in any manner.

### EXAMPLE 1 : Preparation of ginseng extract containing ginsenoside Rg₃

100g of fresh ginseng was introduced into a sealed container and then heated for 2 hours at 130°C. The obtained processed ginseng was extracted with 200ml of methanol to obtain the methanol extract and then methanol was removed from the extract by evaporation. The residue was suspended in 100ml of water, extracted 3 times with 100ml of ether, and then the remaining aqueous layer was extracted 3 times with 100ml of butanol saturated with water to obtain the butanol extract containing saponins. This butanol extract was dried and then subjected to silica gel column chromatography (eluent; ethyl acetate/methanol/water = 20:1:1) to obtain 1.5g of the fraction containing 60% of the desired ginsenoside Rg₃.

### EXAMPLE 2 : Preparation of ginseng extract containing ginsenoside Rg₅

1.0g of a fraction containing 50% of the desired ginsenoside Rg₅ was obtained according to the same procedure as Example 1.

### EXAMPLE 3 : Preparation of ginseng extract containing ginsenoside Rg₃

1kg of dried root-hair ginseng was extracted with 2L of methanol for 4 hours under reflux and then filtered. The ginseng extract thus obtained was dried under reduced pressure. The resulting ginseng extract in the form of a syrup was introduced into an autoclave and then heated for 4 hours at 120°C. The heat-treated ginseng extract was subjected to silica gel column chromatography according to the same method as Example 1 to obtain 20g of the fraction containing 65% of the desired ginsenoside Rg₃.

### EXAMPLE 4 : Preparation of ginseng extract containing ginsenoside Rg₅

10g of a fraction containing 60% of the desired ginsenoside Rg₅ was obtained according to the same procedure as Example 3.

### EXAMPLE 5 : Preparation of ginseng extract containing ginsenoside Rg₃

1kg of white ginseng was extracted with 2L of methanol for 4 hours under reflux and then filtered. The ginseng extract thus obtained was dried under reduced pressure. The resulting ginseng extract in the form of a syrup was treated with an acid according to the known method(see: Yakhak Hoeji, Vol. 35(5), pp 432-437, 1991). Specifically, the ginseng extract was dissolved in 1L of a solvent mixture of water and acetic acid(1:1) and heated for 2 hours at 70°C while stirring. Then, the solvent was removed to obtain the acid-treated ginseng extract, which is subjected to silica gel column chromatography according to the same manner as Example 1 to obtain 20g of the fraction containing 72% of the desired ginsenoside Rg₃.

### EXAMPLE 6 : Preparation of ginseng extract containing ginsenoside Rg₅

10g of a fraction containing 60% of the desired ginsenoside Rg₅ was obtained according to the same procedure as Example 5.

### EXAMPLE 7 : Preparation of processed panaxadiol saponin fraction

1kg of ginseng was extracted with 200ml of methanol to obtain the methanol extract and then methanol was removed from the extract by evaporation. The residue was suspended in 100ml of water and extracted 3 times with 100ml of ether. The remaining aqueous layer was extracted 3 times with a solvent mixture of ethyl acetate and butanol(10:1) and the remaining aqueous layer was extracted again 3 times with 100ml of butanol to obtain the panaxadiol saponin fraction. This panaxadiol saponin fraction was heat-treated for 3 hours at 130°C according to the same manner as Example 1 to obtain about 40g of the processed panaxadiol saponin fraction.

### Example 8 : Preparation of ginsenoside Rg₃

1g of the fraction containing ginsenoside Rg₃ prepared in Example 3 above was repeatedly subjected to silica gel column chromatography according to the same manner as Example 1 using the solvent mixture of ethyl acetate/methanol/water(20:1:1) as an eluent to obtain 500mg of the fraction containing 92% of the desired ginsenoside Rg₃. The fraction containing ginsenoside Rg₃ thus obtained was recrystallized from the solvent mixture of methanol and ethylacetate to obtain about 400mg of the desired ginsenoside Rg₃.

The ginsenoside Rg₃ thus obtained represents ¹³C-NMR spectrum and mass spectrum which coincide with the data known from literature(see: Yakugaku Zasshi, 103(6), pp 612-622, 1983). That is, Rg₃ shows a specific peak in CNMR spectrum when about 10mg of ginsenoside Rg₃ thus obtained is dissolved in 600 µl of pyridine-d₅(99.5%) and then subjected to CNMR spectroscopy. In addition, a molecular ion peak of 785 ([M+H]⁺) or 807([M+Na)⁺) is represented in the mass spectrum(FAB+) of ginsenoside Rg₃.

### Example 9 : Preparation of ginsenoside Rg₅

200mg of a fraction containing about 90% of ginsenoside Rg₅ was obtained according to the same procedure as Example 8 from 1g of the fraction containing Rg₅ prepared in Example 4 above. Then, the fraction thus obtained was recrystallized from a solvent mixture of methanol and ethyl acetate to produce about 150mg of the desired ginsenoside Rg₅.

The ginsenoside Rg₅ thus obtained represents the following characteristics.
MS(FAB+, m/z) : 789 ([M+Na]⁺), 805 ([M+K]⁺)
¹³C-NMR (ppm, pyridine-d₅) : δ 13.0, 16.0, 16.5, 16.6, 17.0, 17.8, 18.5, 25.8, 26.7, 27.0, 27.4, 28.1, 32.3, 32.6, 35.3, 37.0, 39.2, 39.7, 40.2, 50.5, 50.9, 51.2, 56.4, 62.6, 62.8, 71.4, 72.4, 72.6, 77.2, 77.9, 78.1, 78.3, 78.3, 83.5, 88.9, 105.2, 106.1, 123.5, 124.6, 131.2, 140.2

### Test Example 1 : Vasodilation activity of ginsenoside Rg₃ and Rg₅

Vascular endothelium plays a very important role in keeping homeostasis in the body by controlling blood stream and tension of blood vessel and by inhibiting platelet coagulation. Particularly, it is known that the vascular endothelium releases nitric oxide (NO) as an endothelium-derived relaxing factor(EDRF), which acts on smooth muscle of blood vessel to activate soluble guanilate cyclase and to relax the blood vessel by increasing the cyclic GMP in tissue. When the function of endothelium is damaged by circulatory disorders such as hypertension, hypercholesterolemia, etc., the released amount of nitric oxide is decreased to cause the loss of the normal function of controlling blood vessel. However, since most of the saponin destroy the endothelial cells to cause severe side effects such as hemolysis or toxicity against fishes, etc., they are undesirable. Therefore, it has been required a component which exhibits a vasodilation upon blood vessel without any damage to endothelium.

The present inventors have identified that ginsenoside Rg₃ and Rg₅ exhibit a potent endothelium-dependent vasodilation different from the conventional saponin components, which is established by the following experiment.

### 1. Experimental Procedure :

Sprague-Dawley(SD) rats weighing 300-400g were sacrificed and their thoracic aortae were removed quickly, and then placed in a Krebs-Ringer-sodium bicarbonate solution (control solution, NaCl 118.3; KCl 4.7; MgSO₄ 1.2; KH₂PO₄ 1.2; CaCl₂ 2.5; NaHCO₃ 25.0; CaEDTA 0.016 and glucose 11.1mM). Blood within the aortae, adipose and connective tissues surrounding the blood vessel were removed and then the clean aortae were cut down to prepare the aortic rings having 2-3mm length. The aortic rings were suspended vertically in an organ chamber filled with 25ml of control solution (pH 7.4). In some aortic rings, the endothelium was removed mechanically by rolling the rings 7 to 8 times on a tissue towel with pincette. The bottom of aortic ring was fixed to the organ chamber and the top thereof was connected to a transducer coupler through stirrups for recording of isometric tension.

To the aortic rings showing a stable contraction induced by addition of 10⁻⁶M phenylephrine to the organ chamber was added ginsenoside Rg₃ or Rg₅-dimethylsulfoxide solution alone or together with 10⁻⁶M methylene blue(MB) or 10⁻⁶M nitro-L-arginine(NLA) in order to observe the vasodilation thereof. The degree of contraction by 10⁻⁶M phenylephrine was set to 100% and the degree of relaxation caused by ginsenoside Rg₃ or Rg₅ was represented as percent(%) with respect thereto.

### 2. Experimental Result :

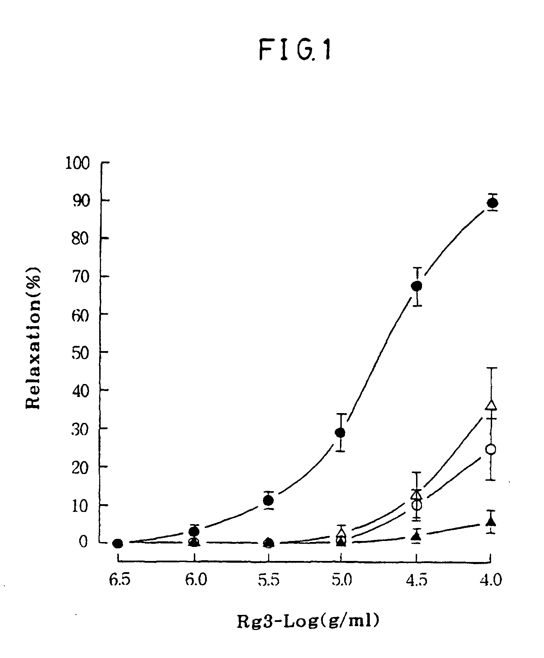

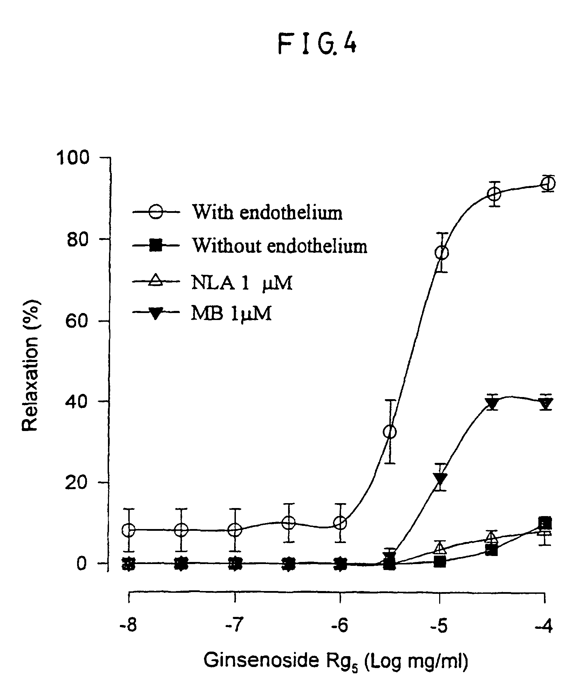

As depicted in Figures 1 and 4, ginsenoside Rg₃ and Rg₅ show a concentration-dependent relaxation to the blood vessel with endothelium. However, they exhibit no effect to the blood vessel without endothelium. Also, the vasodilation of ginsenoside Rg₃ or Rg₅ is significantly inhibited by nitro-L-arginine(NLA), an inhibitor of nitric oxide synthase, or by methylene blue(MB), an inhibitor of soluble guanylate cyclase.

From such results, it can be seen that ginsenoside Rg₃ and Rg₃ exhibit a vasodilation only to the blood vessel with endothelium. Therefore, the results suggest that ginsenoside Rg₃ and Rg₅ are safe medicinal components because they do not exhibit side effects such as hemolysis, toxicity against fishes, etc., contrary to other saponin components destroying the endothelial cells.

### Test Example 2 : Vasodilation activity of the processed saponin fraction

### 1. Experimental Procedure :

Vasodilation of the processed panaxadiol saponin fraction obtained in Example 7 as mentioned above was determined according to the same experimental procedure as Test Example 1.

### 2. Experimental Result :

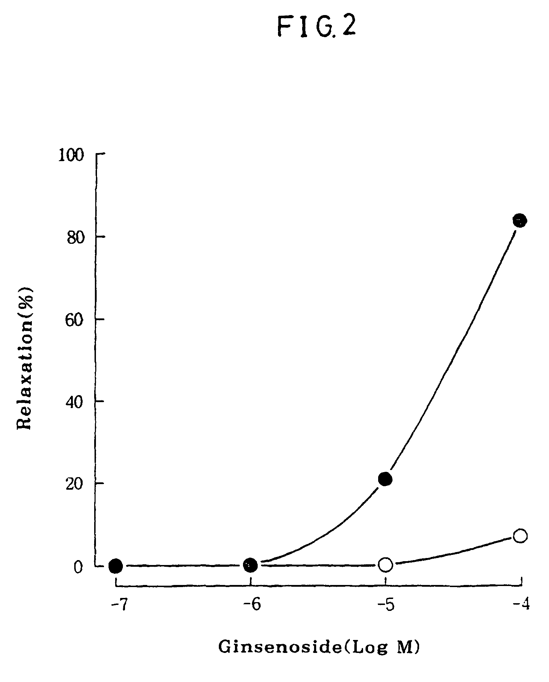

As depicted in Figure 2, the processed panaxadiol saponin fraction shows a concentration-dependent relaxation to the blood vessel with endothelium. However, it exhibits no effect to the blood vessel without endothelium. Also, the vasodilation thereof is significantly inhibited by nitro-L-arginine (NLA), an inhibitor of nitric oxide synthase, or by methylene blue(MB), an inhibitor of soluble guanylate cyclase.

The results show that the processed panaxadiol saponin fraction exhibits a vasodilation only to the blood vessel with endothelium. Therefore, it suggests that the processed panaxadiol fraction is a safe medicinal component because it does not exhibit side effects such as hemolysis, toxicity against fishes, etc., contrary to other saponin components destroying the endothelial cells.

### Test Example 3 : Cytotoxicity of ginsenoside Rg₃

### 1. Experimental Procedure :

Human umbilical vein endothelium cells(HUVEC) cultured in M199 medium were suspended in physiological saline to a concentration of 20000 cells/180µl. The cell suspension was introduced into each well of 96-well plate in an amount of 180µl, and then 20µl of dimethylsulfoxide as a solvent or the same volume of Rg₃ dissolved in dimethylsulfoxide was added thereto. The plate was incubated for 48 hours in a cell-incubator of 37°C while keeping 5% CO₂. Then, 50µl of MTT(3-(4,5-dimethyl-thiazol-2'-yl)-2,5-diphenyltetrazolium bromide) dissolved in phosphate buffered solution in a concentration of 2mg/ml was added thereto and incubated again for 4 hours under the same condition as mentioned above. Thereafter, the supernatant was removed and 200µl of dimethylsulfoxide was added to measure the absorbance at 540nm. The absorbance measured was converted into cell number to determine IC₅₀ of dimethylsulfoxide solvent and Rg₃ which is the concentration to inhibit the cell growth by 50% and can be used as an index of cytotoxicity.

### 2. Experimental Result :

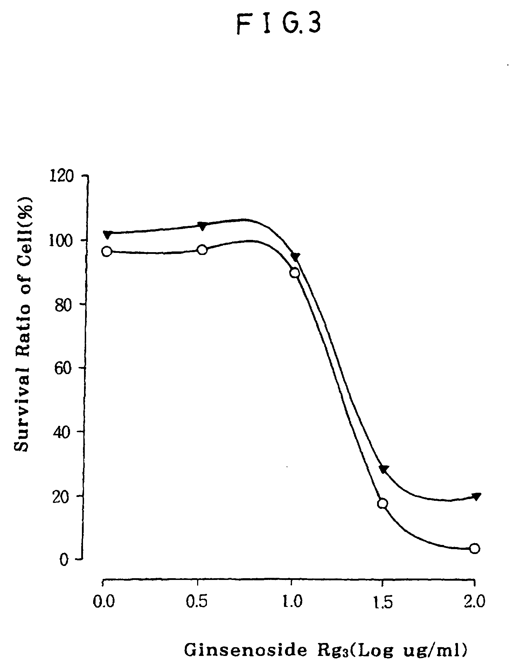

In the case of dimethylsulfoxide (control group), it began to exhibit cytotoxicity from 0.6% and suppressed 100% of the cell growth at 6%. Thus, the cytotoxicity of the solvent depends on its amount and IC₅₀ thereof is 18.4µg/ml. Rg₃ showed the cytotoxity at the concentration of 1 to 100µg/ml in the same manner of results with dimethylsulfoxide. IC₅₀ of Rg₃, however, is 26.7µg/ml which is somewhat higher than that of the solvent, and therefore it is recognized that Rg₃ has lower cytotoxicity than the solvent. That is, since survival ratio of the Rg₃ receiving group is higher than that of control group, it is recognized that Rg₃ has a stimulating effect on cell growth (see, Figure 3).

### Test Example 4 :Cytotoxicity of ginsenoside Rg₅

### 1. Experimental Procedure :

Human umbilical vein endothelium cells(HUVEC) cultured in M199 medium were suspended in physiological saline to a concentration of 20000 cells/180µl. The cell suspension was introduced into each well of 96-well plate in an amount of 180µl, and then 20µl of distilled water as a solvent or the same volume of Rg₅ dissolved in distilled water was added thereto. The plate was incubated for 48 hours in a cell-incubator of 37°C while keeping 5% CO₂. Then, 50µl of MTT(3-(4,5-dimethyl-thiazol-2'-yl)-2,5-diphenyltetrazolium bromide) dissolved in phosphate buffered solution in a concentration of 2mg/ml was added thereto and incubated again for 4 hours under the same condition as mentioned above. Thereafter, the supernatant was removed and 200µl of dimethylsulfoxide was added to measure the absorbance at 540nm.

### 2. Experimental Result :

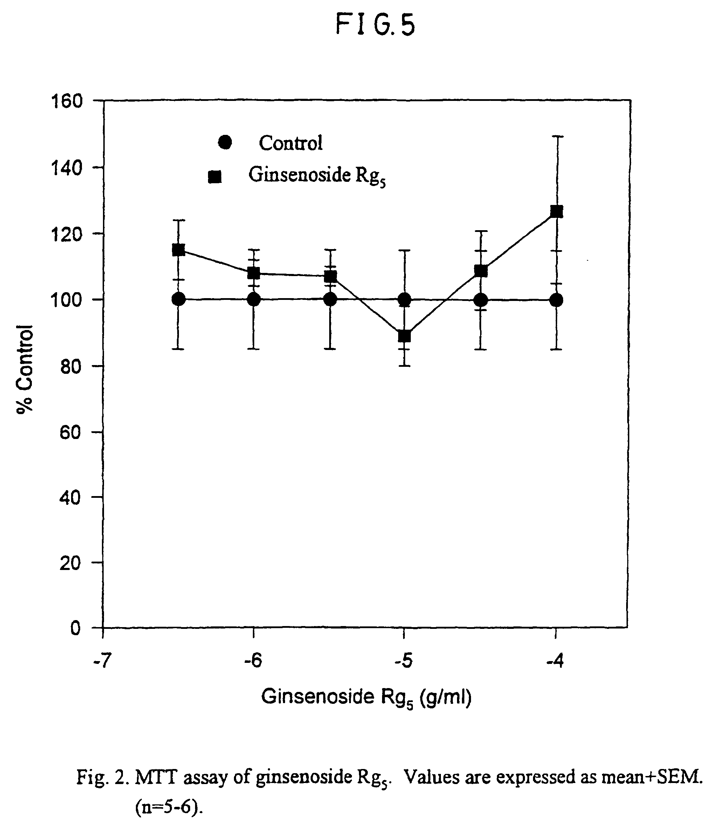

As depicted in Figure 5, the cells with ginsenoside R_{g5} grow more actively than that with distilled water only though there is no statistical significance. Therefore, it can be seen that ginsenoside Rg₅ has no cytotoxicity.

### COMPOSITION EXAMPLES

In the following composition examples, Rg₃ or a fraction containing Rg₃ can be replaced with Rg₅, Rg₃ and Rg₅ or a fraction containing the same, respectively.

### COMPOSITION EXAMPLE 1

| Component | Content |
|---|---|
| Corn starch | 44 g |
| Crystalline cellulose | 40 g |
| Calcium carboxymethylcellulose | 5 g |
| Magnesium stearate | 1 g |
| Fraction obtained in Example 1 | 10 g |
| Total : | 100 g |

According to the composition above, all the components were mixed uniformly and then molded compressively in a tabletting machine to prepare tablets weighing 500mg and containing 50mg of the fraction obtained in Example 1 per each tablet. The daily dosage for an adult, 3 to 10 tablets, can be divided into several portions and administered over several times.

### COMPOSITION EXAMPLE 2

| Component | Content |
|---|---|
| Crystalline cellulose | 34.5 g |
| 10% Hydroxypropylcellulose - ethanol solution | 50 g |
| Calcium carboxymethylcellulose | 5 g |
| Magnesium stearate | 0.5 g |
| Fraction obtained in Example 5 | 10 g |
| Total : | 100 g |

According to the composition above, crystalline cellulose, 10% hydroxypropylcellulose-ethanol solution and the fraction obtained in Example 5 were mixed uniformly, dried by using a granulator according to a conventional manner and then grinded. Calcium carboxymethylcellulose and magnesium stearate were mixed therewith and then the mixture was molded compressively in a tabletting machine to prepare tablets weighing 500mg and containing 50mg of the fraction obtained in Example 5 per each tablet. The daily dosage for an adult, 3 to 10 tablets, can be divided into several portions and administered over several times.

### COMPOSITION EXAMPLE 3

| Component | Content |
|---|---|
| Injectable distilled water | 86.5 g |
| Ethanol | 5 g |
| Soybean phospholipid | 2.5 g |
| Glycerin | 5 g |
| Rg₃ obtained in Example 8 | 1 g |
| Total : | 100 g |

According to the composition above, Rg₃ obtained in Example 8 was dissolved in ethanol and soybean phophoslipid. Then, the resulting solution was emulsified by adding injectable distilled water and glycerin solution to prepare the injectable preparation.

### COMPOSITION EXAMPLE 4

| | Component | Content |
|---|---|---|
| 1) | Pyridoxine hydrochloride | 300 mg |
| 2) | Nicotinic acid amide | 1 g |
| 3) | Calcium pantothenate | 1 g |
| 4) | Riboflavine | 200 mg |
| 5) | Citric acid | 30 g |
| 6) | Fraction obtained in Example 1 | 20 g |
| 7) | Epimedii Herba extract | 200 mg |
| 8) | Water added by | 10 L |

According to the composition above, components 1) to 7) were dissolved in component 8) to prepare the solution. 100ml of this solution contains 200mg of the fraction obtained in Example 1.

### COMPOSITION EXAMPLE 5

| Component | Content |
|---|---|
| Crystalline cellulose | 440 mg |
| Magnesium stearate | 10 g |
| Rg₃ obtained in Example 8 | 50 g |
| Total: | 500 g |

All the components described in above were mixed uniformly, granulated according to a conventional manner and then filled into capsules. Each capsule preparation thus obtained is 500mg in weight and contains 50mg of Rg₃. The daily dosage for an adult, 3 to 10 capsules, can be divided into several portions and administered over several times.

## Claims

1. A process for preparing ginsenoside Rg₃ and / or Rg₅, **characterized in that** the plant of Panax genus is heated for 0.5 to 20 hours at a temperature of 110 to 180°C, the processed ginseng thus obtained is extracted with an organic solvent and then the extract is subjected to chromatography to separate ginsenoside Rg₃.

2. A process for preparing ginsenoside Rg₃ and / or Rg₅, **characterized in that** the plant of Panax genus is extracted with an organic solvent and then the extract is heated for 0.5 to 20 hours at a temperature of 110 to 180°C and then subjected to chromatography to separate ginsenoside Rg₃ and / or Rg₅.

3. A process for preparing ginsenoside Rg₃ and / or Rg₅, **characterized in that** the plant of Panax genus is extracted with an organic solvent and then the extract is treated with an acid at a temperature of 50 to 100°C and then subjected to chromatography to separate ginsenoside Rg₃ and / or Rg₅.

## Patentansprüche

1. Verfahren zur Herstellung von Rg₃- und/oder Rg₅-Ginsenosid, **dadurch gekennzeichnet, dass** die Pflanze der Gattung Panax 0,5 bis 20 Stunden lang auf eine Temperatur von 110 bis 180°C erwärmt, der so erhaltene, aufgearbeitete Ginseng mit einem organischen Lösungsmittel extrahiert und das Extrakt dann einer Chromatografie unterzogen wird, um Rg₃-Ginsenosid abzutrennen.

2. Verfahren zur Herstellung von Rg₃- und/oder Rg₅-Ginsenosid, **dadurch gekennzeichnet, dass** die Pflanze der Gattung Panax mit einem organischen Lösungsmittel extrahiert und das Extrakt dann 0,5 bis 20 Stunden land auf eine Temperatur von 110 bis 180°C erwärmt und dann einer Chromatografie unterzogen wird, um Rg₃- und/oder Rg₅-Ginsenosid abzutrennen.

3. Verfahren zur Herstellung von Rg₃- und/oder Rg₅-Ginsenosid, **dadurch gekennzeichnet, dass** die Pflanze der Gattung Panax mit einem organischen Lösungsmittel extrahiert und das Extrakt dann mit einer Säure bei einer Temperatur von 50 bis 100°C behandelt und dann einer Chromatografie unterzogen wird, um Rg₃- und/oder Rg₅-Ginsenosid abzutrennen.

## Revendications

1. Un traitement de préparation du ginsenoside Rg₃ et/ou Rg₅, **caractérisé en ce que** la plante du genre *Panax* est chauffée pendant 0,5 à 20 heures à une température de 110 à 180°C, le ginseng traité ainsi obtenu subit une extraction par un solvant organique, puis l'extrait est soumis à une chromatographie pour séparer le ginsenoside Rg₃.

2. Un traitement de préparation du ginsenoside Rg₃ et/ou Rg₅, **caractérisé en ce que** la plante du genre *Panax* subit une extraction par un solvant organique, puis l'extrait est chauffé pendant 0,5 à 20 heures à une température de 110 à 180°C, puis soumis à une chromatographie pour séparer le ginsenoside Rg₃ et/ou Rg₅.

3. Un traitement de préparation du ginsenoside Rg₃ et/ou Rg₅, **caractérisé en ce que** la plante du genre *Panax* subit une extraction par un solvant organique, puis l'extrait est traité par un acide à une température de 50 à 100°C, puis soumis à une chromatographie pour séparer le ginsenoside Rg₃ et/ou Rg₅.
